(19) Europäisches Patentamt
European Patent Office
Office européen des brevets

(11) Veröffentlichungsnummer: **0 555 657 A1**

# (12) EUROPÄISCHE PATENTANMELDUNG

(21) Anmeldenummer: **93100649.8**

(22) Anmeldetag: **18.01.93**

(51) Int. Cl.[5]: **C07D 401/10**, C07D 471/04, C07D 491/04, A61K 31/44, A61K 31/50

(30) Priorität: **30.01.92 DE 4202526**

(43) Veröffentlichungstag der Anmeldung:
**18.08.93 Patentblatt 93/33**

(84) Benannte Vertragsstaaten:
**AT BE CH DE DK ES FR GB GR IE IT LI LU MC NL PT SE**

(71) Anmelder: **BAYER AG**

**D-51368 Leverkusen(DE)**

(72) Erfinder: **Straub, Alexander, Dr.**
**Moospfad 30**
**W-5600 Wuppertal 1(DE)**
Erfinder: **Stoltefuss, Jürgen, Dipl.-Ing.**
**Parkstrasse 20**
**W-5657 Haan(DE)**
Erfinder: **Goldmann, Siegfried, Dr.**
**Am Osterholz 91**
**W-5600 Wuppertal 11(DE)**
Erfinder: **Gross, Rainer, Prof. Dr.**
**Platzhofstrasse 23**
**W-5600 Wuppertal 11(DE)**
Erfinder: **Bechem, Martin, Dr.**
**Hans-Böckler-Strasse 102**
**W-5600 Wuppertal 1(DE)**
Erfinder: **Hebisch, Siegbert, Dr.**
**Richard-Seel-Weg 11**
**W-5600 Wuppertal 11(DE)**
Erfinder: **Hütter, Joachim, Dr.**
**Teschensudbergerstrasse 13**
**W-5600 Wuppertal 12(DE)**
Erfinder: **Rounding, Howard-Paul, Dr.**
**Pahlkestrasse 15**
**W-5600 Wuppertal 1(DE)**

(54) **Neue 4-Cinnolinyl- und 4-Naphthyridinyl-dihydropyridine, Verfahren zur ihrer Herstellung und ihre Verwendung in Arzneimitteln.**

(57) Die Erfindung betrifft neue 4-Cinnolinyl- und 4-Naphthyridinyl-dihydropyridine der allgemeinen Formel I

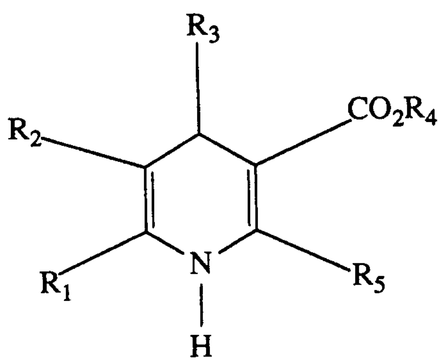

(I),

in welcher $R^1$ bis $R^5$ die in der Beschreibung angegebene Bedeutung haben, Verfahren zu ihrer Herstellung und ihre Verwendung in Arzneimitteln, insbesondere in Mitteln mit positiv inotroper Wirkung.

EP 0 555 657 A1

Die Erfindung betrifft neue 4-Cinnolinyl- und 4-Naphthyridinyl-dihydropyridine,Verfahren zu ihrer Herstellung und ihre Verwendung in Arzneimitteln, insbesondere in Mitteln mit positiv inotroper Wirkung.

Es ist bereits bekannt, daß 1,4-Dihydropyridine gefäßerweiternde Eigenschaften besitzen und als Coronarmittel und Antihypertensiva verwendet werden können [vgl. Brit. Patent 1 173 062 und 1 358 951; DE-OS 2 629 892 und 27 52 820]. Weiterhin ist bekannt, daß 1,4-Dihydropyridine eine Hemmung der Kontraktionskraft von glatten und cardialen Muskeln bewirken und zur Behandlung von Coronar- und Gefäßerkrankungen eingesetzt werden können [vgl. Fleckenstein, Ann. Rev. Pharmacol. Toxicol., 17, 149 - 166 (1977)].

Es ist außerdem bekannt, daß 3-Nitro-dihydropyridine im allgemeinen neben einer positiv inotropen Herzwirkung den Nachteil einer unerwünschten konstringierenden Wirkung an den Koronargefäßen zeigen können [vgl. Schramm et al., Nature 303, 535-537 (1983) und DE-OS 3 447 169].

Bei Kenntnis des Standes der Technik war es nicht vorhersehbar, daß die erfindungsgemäßen Verbindungen eine kontraktionskraftverstärkende, am Herzmuskel positiv inotrope Wirkung mit weitgehend gefäßneutralem bzw. -dilatierendem Verhalten besitzen.

Die Erfindung betrifft 4-Cinnolinyl- und 4-Naphthynridinyl-dihydropyridine der allgemeinen Formel (I),

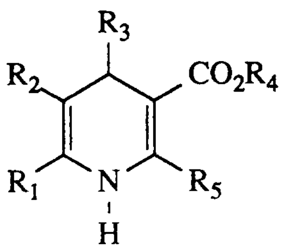

(I),

in welcher

$R^1$ und $R^5$ gleich oder verschieden sind und für geradkettiges oder verzweigtes Alkyl mit bis zu 8 Kohlenstoffatomen stehen,

$R^2$ für geradkettiges oder verzweigtes Alkoxycarbonyl mit bis zu 6 Kohlenstoffatomen steht, das gegebenenfalls durch geradkettiges oder verzweigtes Alkoxy mit bis zu 4 Kohlenstoffatomen substituiert ist,
für Nitro oder Cyano steht,

oder

$R^1$ und $R^2$ gemeinsam einen Lactonring der Formel

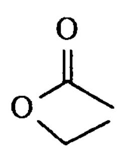

bilden,

$R^3$ für einen heterocyclischen Rest der Formel

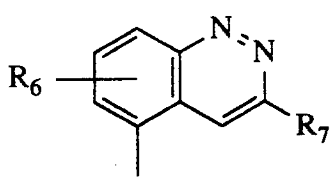

oder

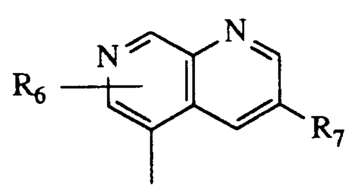

steht,
worin

$R^6$ Wasserstoff, Halogen oder geradkettiges oder verzweigtes Alkyl oder Alkoxy mit jeweils bis zu 8 Kohlenstoffatomen bedeutet,

$R^7$ Aryl mit 6 bis 10 Kohlenstoffatomen bedeutet, das gegebenenfalls bis zu 2-fach gleich oder verschieden durch Halogen, Nitro, Cyano, Trifluormethyl, Trifluormethoxy, Trifluormethylthio, geradkettiges oder verzweigtes Alkyl, Alkoxy oder Alkoxycarbonyl mit jeweils bis zu 8 Kohlenstoffatomen oder Carboxy substituiert ist, oder
Pyridyl oder Thienyl bedeutet,

$R^4$ für Wasserstoff steht, oder
für geradkettiges oder verzweigtes Alkyl, Alkenyl, Alkadienyl oder Alkinyl mit jeweils bis zu 10 Kohlenstoffatomen steht, die gegebenenfalls bis zu 2-fach gleich oder verschieden durch Trifluormethyl, Halogen, Hydroxy, Carboxy, Cyano, Nitro, Phenoxy oder durch geradkettiges oder verzweigtes Alkylthio, Alkoxy, Alkoxycarbonyl, Acyl oder Acyloxy mit jeweils bis zu 8 Kohlenstoffatomen oder durch Phenoxy oder Phenyl substituiert sind, wobei die letzteren ihrerseits bis zu 2-fach gleich oder verschieden durch Halogen oder durch geradkettiges oder verzweigtes Alkyl oder Alkoxy mit jeweils bis zu 6 Kohlenstoffatomen substituiert sein können

und deren physiologisch unbedenklichen Salze.

Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Die erfindungsgemäßen Verbindungen existieren in stereoisomeren Formen, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Bevorzugt sind Verbindungen der allgemeinen Formel (I),
in welcher

$R^1$ und $R^5$ gleich oder verschieden sind und für geradkettiges oder verzweigtes Alkyl mit bis zu 6 Kohlenstoffatomen stehen,

$R^2$ für geradkettiges oder verzweigtes Alkoxycarbonyl mit bis zu 4 Kohlenstoffatomen steht, das gegebenenfalls durch geradkettiges oder verzweigtes Alkoxy mit bis zu 3 Kohlenstoffatomen substituiert ist,
für Nitro oder Cyano steht,

oder

$R^1$ und $R^2$ gemeinsam einen Lactonring der Formel

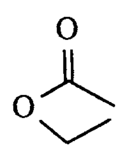

bilden,

$R^3$ für einen heterocyclischen Rest der Formel

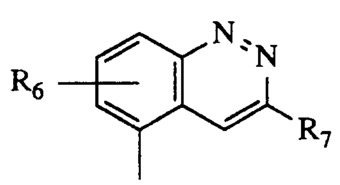

oder

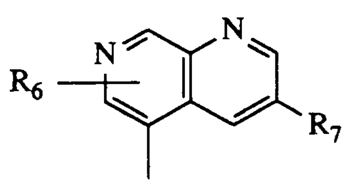

steht,
worin

$R^6$ Wasserstoff, Fluor, Chlor oder geradkettiges oder verzweigtes Alkyl oder Alkoxy mit jeweils bis zu 2 Kohlenstoffatomen bedeutet,

$R^7$ Phenyl bedeutet, das gegebenenfalls durch Fluor, Chlor, Nitro, Cyano, Trifluormethyl oder durch geradkettiges oder verzweigtes Alkyl oder Alkoxy mit jeweils bis zu 6 Kohlenstoffatomen substituiert ist,

$R^4$ für Wasserstoff oder
für geradkettiges oder verzweigtes Alkyl oder Alkenyl mit jeweils bis zu 8 Kohlenstoffatomen steht, die gegebenenfalls durch Trifluormethyl, Fluor, Chlor, Hydroxy, Carboxy, Cyano, Nitro oder durch geradkettiges oder verzweigtes Alkylthio, Alkoxy, Alkoxycarbonyl, Acyl oder Acyloxy mit jeweils bis zu 6 Kohlenstoffatomen oder durch Phenoxy oder Phenyl substituiert sind

und deren physiologisch unbedenklichen Salze.

Besonders bevorzugt sind Verbindungen der allgemeinen Formel (I),
in welcher

$R^1$ und $R^5$ gleich oder verschieden sind und für geradkettiges oder verzweigtes Alkyl mit bis zu 4 Kohlenstoffatomen stehen,

$R^2$ für geradkettiges oder verzweigtes Alkoxycarbonyl mit bis zu 3 Kohlenstoffatomen oder Methoxyethoxycarbonyl steht, oder
für Nitro oder Cyano steht,

oder

$R^1$ und $R^2$ gemeinsam einen Lactonring der Formel

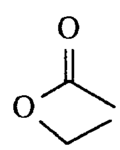

bilden,

$R^3$ für einen heterocyclischen Rest der Formel

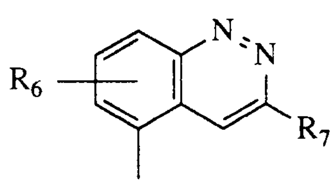

oder

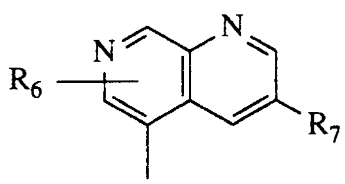

steht,
worin

$R^6$ Wasserstoff, Chlor oder Methyl bedeutet,

$R^7$ Phenyl bedeutet, das gegebenenfalls durch Fluor, Chlor, Nitro, Trifluormethyl oder durch geradkettiges oder verzweigtes Alkyl oder Alkoxy mit jeweils bis zu 4 Kohlenstoffatomen substituiert ist,

$R^4$ für Wasserstoff steht, oder
für geradkettiges oder verzweigtes Alkyl mit bis zu 6 Kohlenstoffatomen steht, das gegebenenfalls durch Trifluormethyl, Hydroxy, Carboxy, Cyano oder durch geradkettiges oder verzweigtes Alkoxycarbonyl, Alkoxy oder Acyloxy mit jeweils bis zu 4 Kohlenstoffatomen substituiert ist

und deren physiologisch unbedenklichen Salze.

Außerdem wurde ein Verfahren zur Herstellung der erfindungsgemäßen Verbindungen der allgemeinen Formel (I) gefunden, dadurch gekennzeichnet,
daß man im Fall, daß $R^1$ und $R^2$ die oben angegebene Bedeutung haben, aber nicht gemeinsam einen

Lactonring bilden,

[A] Aldehyde der allgemeinen Formel (II)

$R^3$-CHO (II),

in welcher

$R^3$ die oben angegebene Bedeutung hat,

zunächst mit Acetessigestern der allgemeinen Formel (III)

$R^5$-CO-$CH_2$-$CO_2$-$R^4$ (III),

in welcher

$R^4$ und $R^5$ die oben angegebene Bedeutung haben,

gegebenenfalls unter Isolierung der entsprechenden Ylidenverbindungen der allgemeinen Formel (IV)

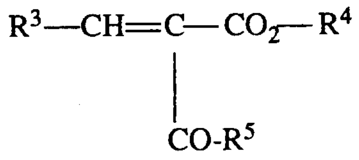

(IV),

in welcher

$R^3$, $R^4$ und $R^5$ die oben angegebene Bedeutung haben,

umsetzt, und anschließend entweder mit Verbindungen der allgemeinen Formel (V)

$R^1$-CO-$CH_2$-$R^2$ (V),

in welcher

$R^1$ und $R^2$ die oben angegebene Bedeutung haben,

in Anwesenheit von Ammoniak oder Ammoniumsalzen, oder direkt mit Aminoderivaten der allgemeinen Formel (VI)

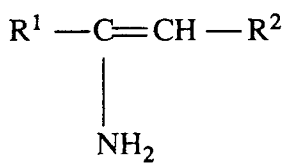

(VI),

in welcher

$R^1$ und $R^2$ die oben angegebene Bedeutung haben,

gegebenenfalls in Gegenwart von inerten organischen Lösemitteln umsetzt,

oder

[B] die Aldehyde der allgemeinen Formel (II) zunächst mit den Verbindungen der allgemeinen Formel (V), gegebenenfalls unter Isolierung der Ylidenverbindungen der allgemeinen Formel (VII)

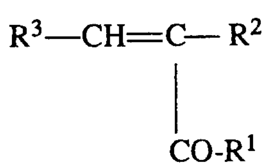

(VII),

in welcher

$R^1$, $R^2$ und $R^3$ die oben angegebene Bedeutung haben,

umsetzt und in einem nächsten Schritt mit den oben aufgeführten Verbindungen der allgemeinen Formel (III) in inerten Lösemitteln, in Anwesenheit von Ammoniak oder Ammoniumsalzen oder direkt mit Enaminocarbonsäurederivaten der allgemeinen Formel (VIII)

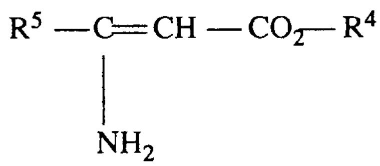

(VIII),

in welcher

$R^4$ und $R^5$ die oben angegebene Bedeutung haben,

umsetzt,

oder im Fall, daß $R^1$ und $R^2$ gemeinsam einen Lactonring bilden,

[C] zunächst nach denen unter [A] und [B] aufgeführten Methoden, Verbindungen der allgemeinen Formel (Ia)

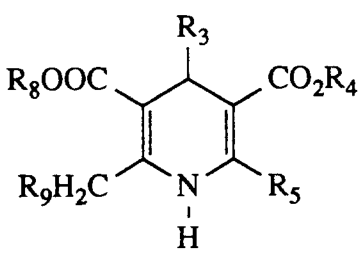

(Ia),

in welcher

$R^3$, $R^4$ und $R^5$ die oben angegebene Bedeutung haben,

$R^8$ für einen $C_1$-$C_6$-Alkyl-Rest steht

und

$R^9$ für eine Abgangsgruppe wie beispielsweise Chlor oder Acetoxy steht,

herstellt und nach bekannten Verfahren einen säure- oder basenkatalysierten Ringschluß anschließt,

oder im Fall, daß $R^4$ nicht Wasserstoff bedeutet,

[D] Verbindungen der allgemeinen Formel (I), in welcher $R^1$, $R^2$, $R^3$, $R^4$ und $R^5$ die oben angegebene Bedeutung haben und $R^4$ für Wasserstoff steht, gegebenenfalls über ein reaktives Säurederivat, mit den entsprechenden Alkoholen umsetzt, wobei durch Einsatz der enantiomerenreinen Carbonsäuren ($R^4$ = H) die entsprechenden Enantiomere der Ester erhalten werden.

Die erfindungsgemäßen Verfahren können durch folgendes Formelschema beispielhaft erläutert werden:

[A]

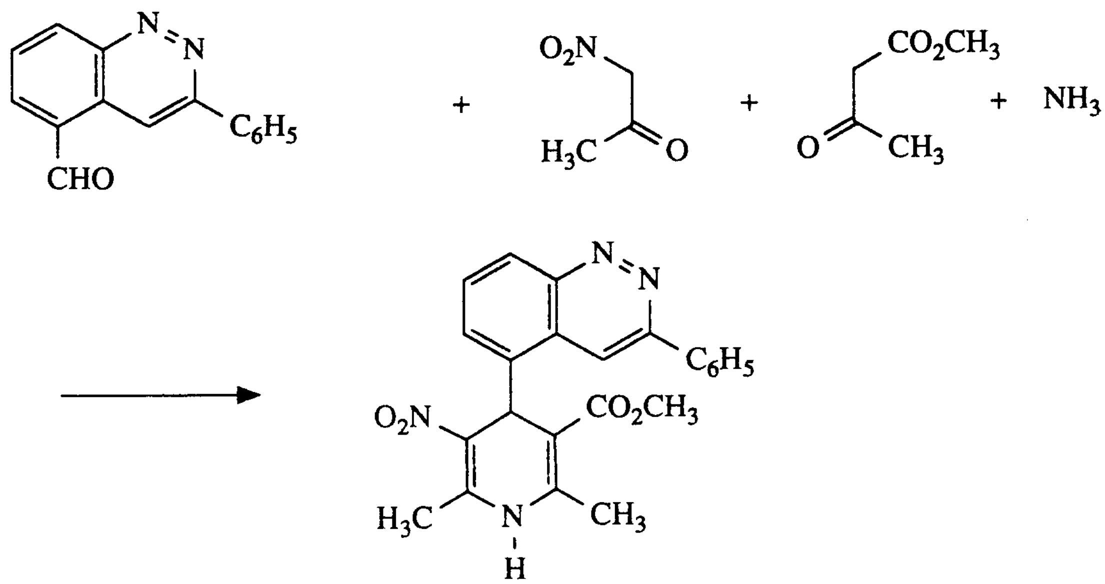

[A]

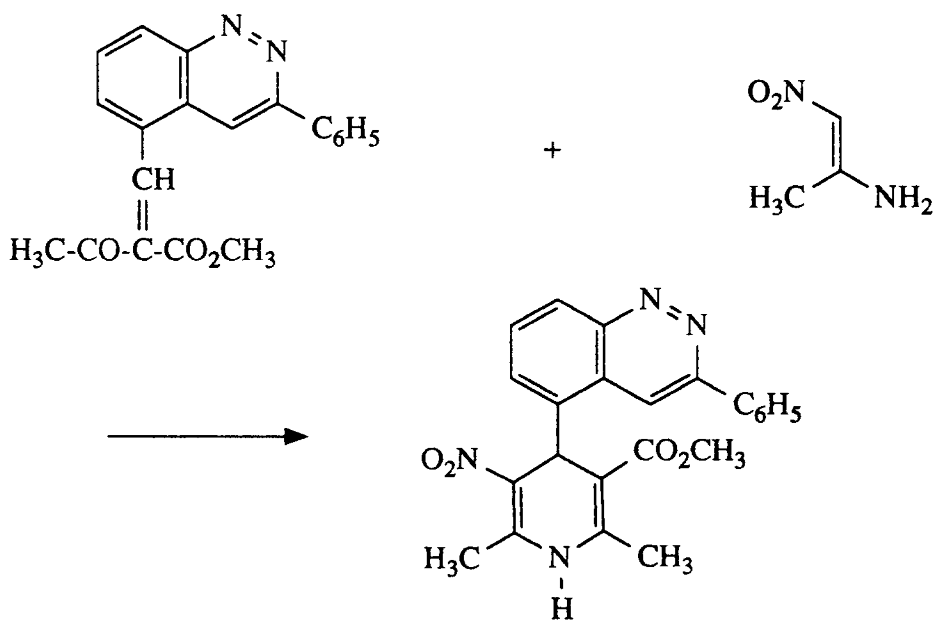

[B]

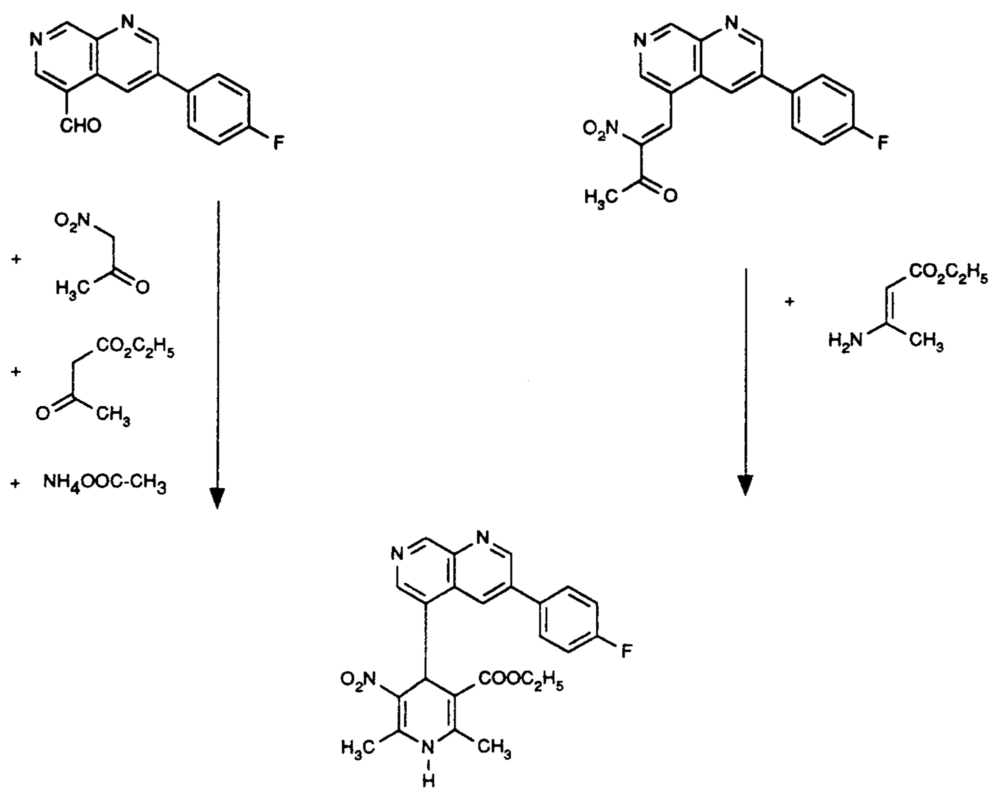


[C]

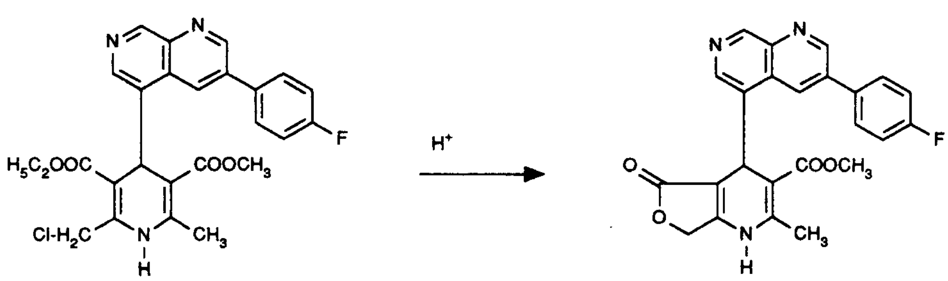


Als Lösemittel eignen sich für die Verfahren [A], [B] und [C] alle inerten organischen Lösemittel. Hierzu gehören bevorzugt Alkohole wie Methanol, Ethanol, n- bzw. iso-Propanol, Ether wie Diethylether, Tetrahydrofuran, Dioxan oder Glykolmono- oder dimethylether, Eisessig, Pyridin, Dimethylformamid, Dimethylsulfoxid, Acetonitril oder Hexamethylphosphorsäuretriamid oder Toluol.

Als Lösemittel für das Verfahren [D] eignen sich die oben aufgeführten Lösemittel mit Ausnahme der Alkohole.

Die Reaktionstemperatur für die Verfahren [A], [B], [C] und [D] können in einem größeren Bereich variiert werden. Im allgemeinen arbeitet man in einem Bereich von 10 °C bis 200 °C, bevorzugt von 20 °C bis 150 °C.

Die Verfahren können bei Normaldruck, erhöhtem oder erniedrigtem Druck (beispielsweise von 0,5 bis 5 bar), vorzugsweise bei Normaldruck durchgeführt werden.

Bei der Durchführung der erfindungsgemäßen Verfahren ist das Verhältnis der an der Reaktion beteiligten Stoffe beliebig. Im allgemeinen arbeitet man jedoch mit molaren Mengen der Reaktanden.

Zur Aktivierung der Carbonsäure eignen sich die üblichen Reagenzien wie anorganische Halogenide, beispielsweise Thionylchlorid, Phosphortrichlorid oder Phosphorpentachlorid, oder Carbonyldiimidazol, Carbodiimide wie Cyclohexylcarbodiimid oder 1-Cyclohexyl-3-[2-(N-methylmorpholino)ethyl]-carbodiimid-p-toluolsulfonat oder N-Hydroxyphthalimid oder N-Hydroxy-benztriazol.

Enantiomerenreine Formen erhält man z.B., indem man Diastereomerengemische der Verbindungen der allgemeinen Formel (I), in welcher $R^4$ für einen optischen Esterrest steht, nach üblicher Methode trennt, anschließend die enantiomerenreinen Carbonsäuren herstellt und dann gegebenenfalls durch Veresterung mit entsprechenden Alkoholen in die enantiomerenreinen Dihydropyridine überführt.

Geeignet als chirale Esterreste sind alle Ester enantiomerenreiner Alkohole wie beispielsweise 2-Butanol, 1-Phenylethanol, Milchsäure, Milchsäureester, Mandelsäure, Mandelsäureester, 2-Aminoalkohole, Zuckerderivate und viele andere enantiomerenreine Alkohole mehr.

Die Trennung der Diastereomeren erfolgt im allgemeinen entweder durch fraktionierte Kristallisation, durch Säulenchromatographie oder durch Craig-Verteilung. Welches das optimale Verfahren ist, muß von Fall zu Fall entschieden werden, manchmal ist es auch zweckmäßig, Kombinationen der einzelnen Verfahren zu benutzen. Besonders geeignet ist die Trennung durch Kristallisation oder Craig-Verteilung bzw. eine Kombination beider Verfahren.

Die Veresterung der enantiomerenreinen Dihydropyridine erfolgt bevorzugt in Ethern wie Diethylether oder Tetrahydrofuran, Dimethylformamid, Methylenchlorid, Chloroform, Acetonitril oder Toluol.

Die Aldehyde der allgemeinen Formel (II) sind ebenfalls neu und können hergestellt werden, indem man, im Fall, daß $R^3$ für den Rest der Formel

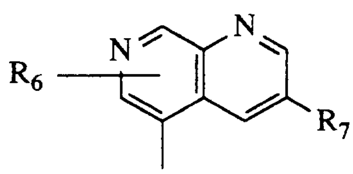

steht,

a) substituierte Pyridine der allgemeinen Formel (IX)

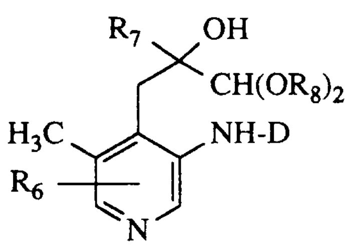

(IX),

in welcher

$R^6$ die oben angegebene Bedeutung hat, vorzugsweise für Chlor steht,

$R^7$ die oben angegebene Bedeutung hat,

D für eine Schutzgruppe wie beispielsweise tert.Butylcarbonyl steht

und

$R^8$ für geradkettiges oder verzweigtes Alkyl mit bis zu 4 Kohlenstoffatomen steht,

zunächst mit Protonensäuren, vorzugsweise Salzsäure und anschließender Hydrierung zu den Verbindungen der allgemeinen Formel (X)

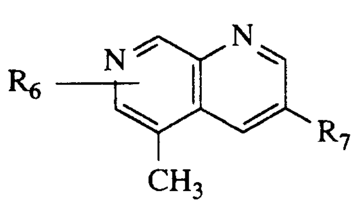

(X),

in welcher

$R^6$ und $R^7$ die oben angegebene Bedeutung haben,

in inerten Lösemitteln cyclisiert und in einem letzten Schritt in einem organischen Lösemittel oder Naphthalin, vorzugsweise Naphthalin, die Methylgruppe oxidiert
und im Fall, daß $R^3$ für den Rest der Formel

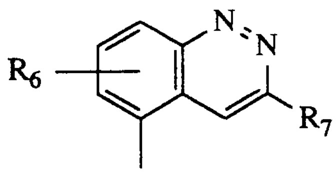

steht,
b) Verbindungen der allgemeinen Formel (XI)

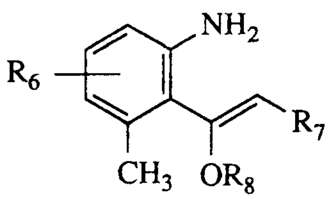

(XI),

in welcher
$R^6$, $R^7$ und $R^8$ die oben angegebene Bedeutung haben,
nach Hydrolyse, über die diazotierte Stufe ($NH_2$ -> $N_2^+$) zu Verbindungen der allgemeinen Formel (XII)

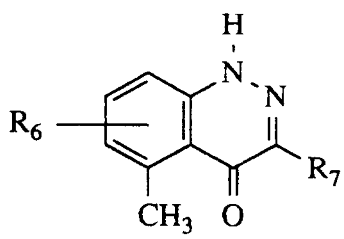

(XII),

in welcher
$R^6$ und $R^7$ die oben angegebene Bedeutung haben,
cyclisiert, in einem zweiten Schritt mit $PCl_5/POCl_3$ in Verbindungen der allgemeinen Formel (XIII)

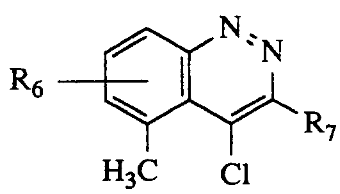

(XIII),

in welcher
$R^6$ und $R^7$ die oben angegebene Bedeutung haben
überführt, hydriert und abschließend die Methyl-Gruppe in inerten Lösemitteln oxidiert.

Die erfindungsgemäßen Verfahren können durch folgendes Formelschema beispielhaft erläutert werden:
a)

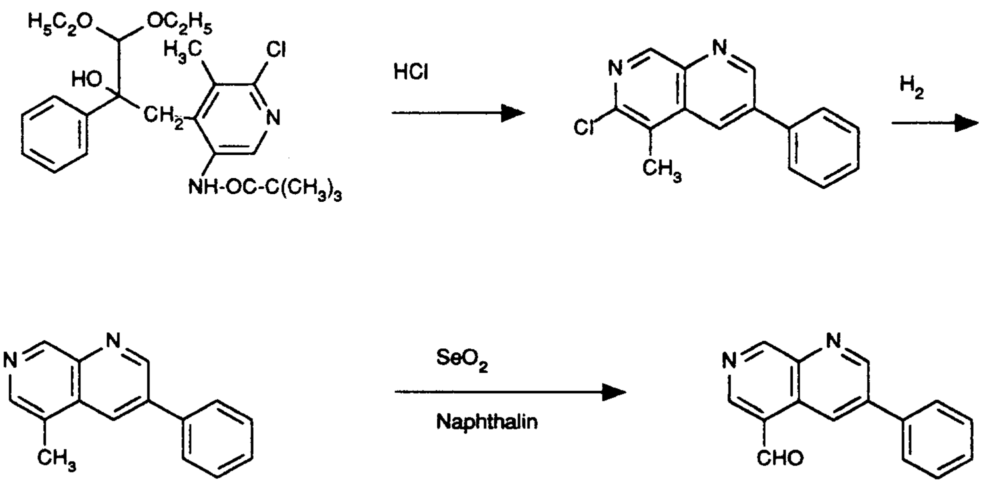


b)

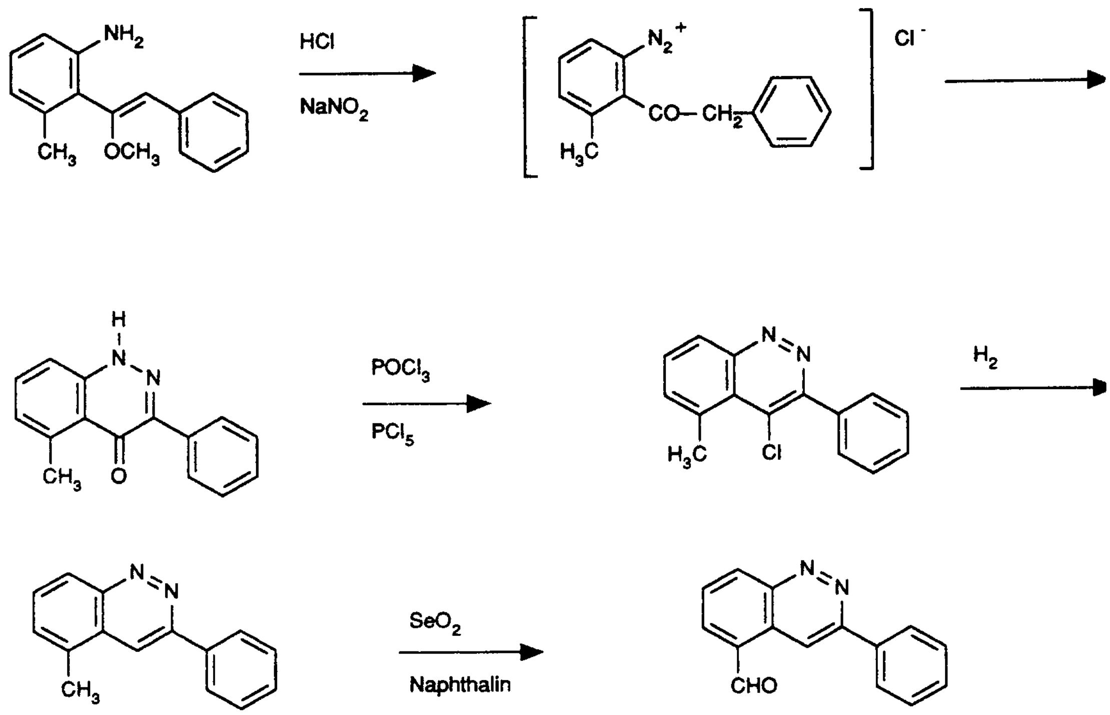


Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether oder Amide wie Hexamethylphosphorsäuretriamid oder Dimethylformamid, oder Essigsäure sowie Methylenchlorid, Tetrachlorkohlenstoff oder Toluol. Ebenso ist es möglich, Gemische der genannten Lösemittel zu verwenden.

Die Oxidation der Verbindungen der allgemeinen Formeln (X) und (XII) erfolgt im allgemeinen mit Oxidationsmitteln wie beispielsweise Chromylchlorid, Cerammoniumnitrat, Silber(II)oxid, Selendioxid oder ein Chrom(VI)oxid in Verbindung mit Essigsäureanhydrid. Bevorzugt ist Selendioxid.

Die Oxidation kann bei Normaldruck oder erhöhtem oder erniedrigtem Druck (beispielsweise von 0,5 bis 5 bar), vorzugsweise bei Normaldruck, durchgeführt werden.

Als Basen eignen sich für einzelne Verfahrensstufen die oben aufgeführten, vorzugsweise Natriumhydroxid und Natriumhydrogencarbonat.

Die Verbindungen der allgemeinen Formeln (X), (XII) und (XIII) sind neu und können nach dem oben aufgeführten Verfahren hergestellt werden.

Die Verbindungen der allgemeinen Formel (IX) sind neu und können hergestellt werden, indem man z.B. die bekannte Verbindung 2-Chlor-3,4-dimethyl-5-nitropyridin [vgl. Y. Morisawa et al., J. Med. Chem. 21, 194 (1978)], zunächst zu der entsprechenden 5-Aminogruppe nach üblichen Methoden, beispielsweise durch Hydrierung mit $H_2$/Pd/C in Dioxan, reduziert, anschließend durch Umsetzung mit Pivaloylchlorid die Aminogruppe blockiert, intermediär mit n-Butyllithium in Tetrahydrofuran deprotoniert und im einem letzten Schritt mit, 2,2-Dialkoxyacetophenonen umsetzt.

Die Verbindungen der allgemeinen Formel (XI) sind bekannt oder können nach üblichen Methoden hergestellt werden.

Die Acetessigester der Formel (III) sind bekannt oder können nach üblichen Methoden hergestellt werden [vgl. D. Borrmann, "Umsetzung von Diketonen mit Alkoholen, Phenolen und Mercaptanen", in Houben-Weyl, Methoden der organischen Chemie, Vol. VIII/4, 230 ff. (1968)].

Die Ylidenverbindungen (IV) und (VII) sind neu, können aber nach üblichen Methoden hergestellt werden [vgl. H. Dornow und W. Sasssenberg, Liebigs Ann. Chem. 602, 14 (1957)].

Die Aminocrotonsäurederivate der Formeln (VI) und (VIII) sind bekannt oder können nach bekannten Methoden hergestellt werden [S.A. Glickman, A.C. Cope, J. Am. Chem. Soc. 67, 1017 (1946)].

Die Verbindungen der allgemeinen Formel (V) sind ebenfalls bekannt [vgl. N. Levy, C.W. Scaife, J. Chem. Soc. (London) 1946, 1100; C.D. Hurd, M.E. Nilson, J. Org. Chem. 20, 927 (1955)].

Die vorstehenden Herstellungsverfahren sind lediglich zur Verdeutlichung angegeben. Die Herstellung der Verbindungen der Formeln (I) und (II) ist nicht auf diese Verfahren beschränkt, sondern jede Modifikation dieser Verfahren ist in gleicher Weise für die Herstellung der erfindungsgemäßen Verbindungen anwendbar.

Die erfindungsgemäßen Verbindungen zeigen ein nicht vorhersehbares, wertvolles pharmakologisches Wirkspektrum. Sie beeinflussen die Kontraktionskraft des Herzens und den Tonus der glatten Muskulatur. Sie können deshalb in Arzneimitteln zur Beeinflussung des pathologisch veränderten Blutdrucks, als Koronartherapeutika und zur Behandlung der Herzinsuffizienz eingesetzt werden. Darüber hinaus können sie zur Behandlung von Herzrhythmusstörungen, zur Senkung des Blutzuckers, zur Abschwellung von Schleimhäuten und zur Beeinflussung des Salz- und Flüssigkeitshaushaltes verwendet werden.

Die Herz- und Gefäßwirkungen wurden am isoliert perfundierten Herzen des Meerschweinchens gefunden. Dazu werden die Herzen von 250 bis 350 g schweren Meerschweinchen verwendet. Die Tiere werden mit einem Schlag auf den Kopf getötet, der Thorax geöffnet, und in die freipräparierte Aorta eine Metallkanüle eingebunden. Das Herz wird mit den Lungen aus dem Thorax herausgetrennt und über eine Aortenkanüle an die Perfusionsapparatur bei laufender Perfusion angeschlossen. Die Lungen werden an den Lungenwurzeln abgetrennt. Als Perfusionsmedium dient eine Krebs-Henseleit-Lösung (1) (118,5 mmol/l NaCl, 4,75 mmol/l KCl, 1,19 mmol/l $KH_2PO_4$, 1,19 mmol/l $MgSO_4$, 25 mmol/l $NaHCO_3$, 0,013 mmol/l $Na_2$EDTA), deren $CaCl_2$-Gehalt 1,2 mmol/l beträgt. Als energielieferndes Substrat werden 10 mmol/l Glucose zugesetzt. Vor der Perfusion wird die Lösung partikelfrei filtriert. Die Lösung wird mit Carbogen (95% $O_2$, 5% $CO_2$) zur Aufrechterhaltung des pH-Wertes 7,4 begast. Die Herzen werden mit konstantem Fluß (10 ml/min) bei 32 °C mittels einer Rollenquetschpumpe perfundiert.

Zur Messung der Herzfunktion wird ein flüssigkeitsgefüllter Latexballon, der über eine Flüssigkeitssäule mit einern Druckaufnehmer verbunden ist, durch den linken Vorhof in den linken Ventrikel eingeführt, und die isovolumetrischen Kontraktionen auf einem Schnellschreiber registriert (Opie, L., J. Physiol. 180 (1965), 529 -541). Der Perfusionsdruck wird mittels eines Druckaufnehmers, der vor dem Herzen mit dem Perfusionssystem in Verbindung steht, registriert. Unter diesen Bedingungen zeigt eine Senkung des Perfusionsdrucks eine Koronardilatation, eine Zu- bzw. Abnahme der linksventrikulären Kontraktionsamplitude eine Senkung bzw. einen Anstieg der Herzkontraktilität an. Die erfindungsgemäßen Verbindungen werden in geeigneten Verdünnungen in das Perfusionssystem kurz vor dem isolierten Herzen perfundiert.

Substanzeffekte auf die Kontraktionsamplitude isolierter Meerschweinchen-Herzvorhöfe bei einer Wirkstoffkonzentration von $10^{-4}$ g/l.

| Bsp.-Nr. | %-Änderung der Ventrikeldruckamplitude |
|---|---|
| 8 | +10 |
| 15 | +35 |
| 23 | +28 |

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral oder parenteral, insbesondere perlingual oder intravenös.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Ausgangsverbindungen

Beispiel 1

3-[2-Chlor-3-methyl-5-pivaloylamino-4-pyridyl]-1,1-diethoxy-2-hydroxy-2-phenylpropan

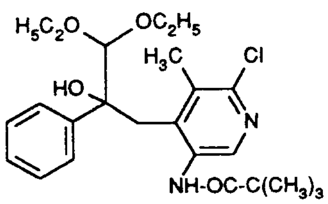

144,3 g (0,6 mol) 2-Chlor-3,4-dimethyl-5-pivaloylaminopyridin werden in 2,9 l abs. THF gelöst und unter Argon bei -78 °C mit 127 ml einer 10,4 N Lösung von n-BuLi in n-Hexan (1,32 mmol) versetzt. Man rührt 3 h bei 0 °C, gibt langsam bei -78 °C 129,8 ml (0,6 mol) 2,2-Diethoxyacetophenon hinzu und läßt bei Raumtemperatur über Nacht rühren. Dann wird der Ansatz in Wasser gegeben und 3 mal mit Essigester extrahiert, getrocknet und einrotiert. Für die weitere Umsetzung ist die Reinheit ausreichend. Nach Chromatographie erhält man 180,5 g (67%) der Titelverbindung.
Mp.: 133 °C

Beispiel II

6-Chlor-5-methyl-3-phenyl-1,7-naphthyridin

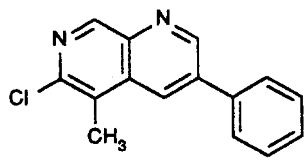

180,5 g (0,4 mol) fein verteilte Verbindung aus Beispiel I werden in 2,6 l 2N HCl 5 h unter Rückfluß mit einem mechanischen Rührwerk intensiv gerührt. Anschließend wird mit $NaHCO_3$ neutralisiert, mehrmals mit Essigester extrahiert und getrocknet. Durch Kristallisation erhält man 37,6 g der Titelverbindung. Die Chromatographie der Mutterlauge (Kieselgel, Toluol -> Toluol/Essigester 2:1) liefert weitere 23,14 g der Titelverbindung.
Ausbeute gesamt: 60%
Mp.: 136 °C

Beispiel III

5-Methyl-3-phenyl-1,7-naphthyridin

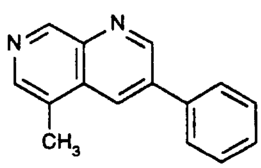

33,7 g (132 mmol) der Verbindung aus Beispiel II werden in 600 ml Ethanol gelöst, mit 293 ml 1N NaOH und 10 g 5% Pd/C versetzt und umgehend in der Parr-Apparatur bei 3 bar für 30 min hydriert. Man filtriert über Kieselgur und verdampft den Ethanolanteil im Vakuum. Das hierbei ausgefallene Produkt wird abgesaugt, mit viel Wasser und dann mit Ether nachgewaschen und getrocknet.
Ausbeute: 23,6 g (81,2%)
Mp.: 135-138 °C

Beispiel IV

3-Phenyl-1,7-naphthyridin-5-carboxaldehyd

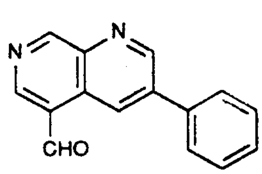

36,2 g (164 mmol) der Verbindung aus Beispiel III werden bei 180 °C in 130 g Naphthalin mit 27,4 g Selendioxid gut gerührt. Nach 5 h gibt man nochmals 3,65 g $SeO_2$ hinzu und rührt weitere 3 h bei 200 °C.

Man läßt abkühlen, löst in $CH_2Cl_2$ und rotiert nach Zugabe von 200 g Kieselgel ein. Den Rückstand trägt man auf eine große Kieselgelsäule auf und chromatographiert (Toluol -> Essigester).
Ausbeute: 18 g (47%)
Mp.: 148 ° C

Beispiel V

1-(1-Amino-3-methyl-2-phenyl)-1-methoxy-2-phenylethen

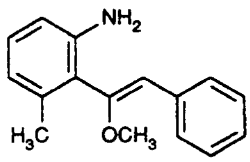

Man erhitzt eine Mischung von 282,3 g (1,21 mol) 2-Iod-3-methylanilin, 420 ml Butyronitril, 147,7 g (1,1 mol) Styrylmethylether, 170 ml Triethylamin und 2,5 g (11 mmol) Pd(II)acetat 8 h lang zum Rückfluß. Die Lösung wird teilweise einrotiert, mit Wasser versetzt und mit Essigester 3 mal extrahiert. Der nach Eindampfen der organischen Phase erhaltene Rückstand wird anschließend auf Kieselgel chromatographiert.
Ausbeute: 158,8 g (60,3%)
MS (EI): 239 (18%), 135 (28%), 91 (22%), 75 (100%)

Beispiel VI

4-Hydroxy-5-methyl-3-phenylcinnolin

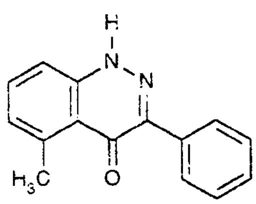

60,5 g (253 mmol) der Verbindung aus Beispiel V werden in 1,1 l 2N HCl 2 h gut gerührt und unter Rückfluß gekocht. Der dabei ausgefallene Feststoff wird ohne Isolierung weiterverarbeitet,indem man die Lösung auf 0 ° C abkühlt und unter heftigem Rühren eine Lösung von 17,4 g (252 mmol) Natriumnitrit in 114 ml Wasser langsam zugibt. Danach läßt man langsam auf Raumtemperatur kommen und rührt weitere 3 Tage. Der Feststoff wird abgesaugt und mit Ether gewaschen.
Ausbeute: 27,3 g (46%)
Mp.: 215-217 ° C

Beispiel VII

4-Chlor-5-methyl-3-phenylcinnolin

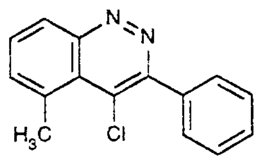

27,3 g (0,116 mol) der Verbindung aus Beispiel VI werden 18 h lang mit 18,3 g $PCl_5$ und 200 ml $POCl_3$ unter Rückfluß gekocht. Man gibt unter gutem Rühren auf Eis, extrahiert mit Methylenchlorid, wäscht die organische Phase mit gesättigter $NaHCO_3$-Lösung, trocknet und verdampft im Vakuum. Man erhält 12 g (41% Rohausbeute) eines Feststoffs, der nach Chromatographie einen Mp. von 128 °C besitzt.

Beispiel VIII

5-Methyl-3-phenylcinnolin

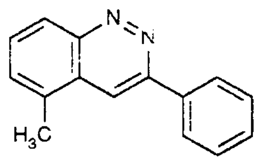

12 g (47,2 mmol) der Verbindung aus Beispiel VII werden in 400 ml Dioxan und 75 ml 1N NaOH in Gegenwart von 1,5 g Pd-C (5%ig) unter DC-Kontrolle mehrere Stunden bei 3 bar in der Parr-Apparatur hydriert. Anschließend wird über Kieselgur abgesaugt und einrotiert. Nach Chromatographie auf Kieselgel (Toluol -> Toluol/Essigester 1:1) erhält man 1,8 g (17%) der reinen Titelverbindung.
Mp.: 101 °C

Beispiel IX

3-Phenyl-5-cinnolincarboxaldehyd

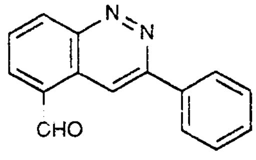

1,65 g (7,5 mmol) der Verbindung aus Beispiel VIII werden in 17 g Naphthalin mit 1,85 g Selendioxid 6 h bei 200 °C gerührt. Nach Chromatographie auf Kieselgel erhält man 0,75 g (43%) der Titelverbindung. Bei manchen Ansätzen ist es notwendig, vor dem vollständigem Umsatz des Edukts die Reaktion abzubrechen, da zunehmend Nebenprodukte auftreten.
Mp.: 156-157 °C

Herstellungsbeispiele

Beispiel 1

5-Cyano-1,4-dihydro-2,6-dimethyl-4-(3-phenylcinnolin-5-yl)pyridin-3-carbonsäureisopropylester

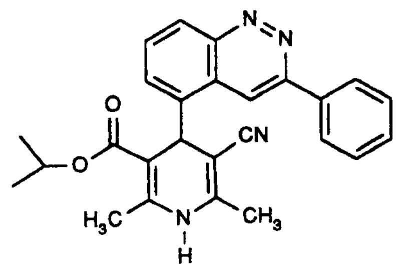

750 mg (3,2 mmol) 3-Phenyl-5-cinnolincarboxaldehyd, 263 mg (3,2 mmol) 3-Aminocrotonsäurenitril und 457 mg (3,2 mmol) Acetessigsäureisopropylester werden in 25 ml Ethanol 2,5 Tage lang unter Rückfluß gekocht. Es wird eingeengt und über eine Kieselgelsäule mit Toluol/Essigsäureethylestergemischen getrennt. Die gewünschten Fraktionen werden gesammelt und eingeengt. Der erhaltene Eindampfrückstand wird mit Ether kristallisiert und abgesaugt. Man erhält 320 mg Kristalle vom Schmp. 200 - 202 °C.

Beispiel 2

5-Cyano-1,4-dihydro-2,6-dimethyl-4-(3-phenyl-1,7-naphthyridin-5-yl)pyridin-3-carbonsäureisopropylester

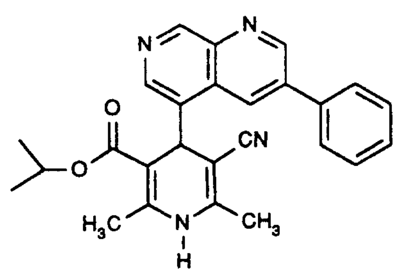

2 g (8,54 mmol) 3-Phenyl-1,7-naphthyridin-5-carboxaldehyd, 701 mg (8,54 mmol) 3-Aminocrotonsäurenitril und 1,22 g (8,54 mmol) Acetessigsäureisopropylester werden in 25 ml Ethanol 3 Tage unter Rückfluß gekocht. Es wird eingeengt und über eine Kieselgelsäule mit Toluol/Essigsäureethylester-Gemischen getrennt. Die gewünschten Fraktionen werden gesammelt und eingeengt. Der erhaltene Eindampfrückstand wird mit Ether kristallisiert und abgesaugt. Man erhält 573 mg Kristalle vom Schmp. 192 °C.

In Analogie zur Vorschrift des Beispiels 1 werden die in Tabelle 1 aufgeführten Verbindungen hergestellt:

Tabelle 1:

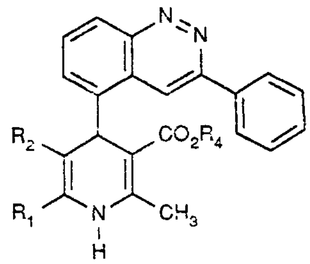

| Bsp.-Nr. | $R^1$ | $R^2$ | $R^4$ | F°C / $R_f$ / Enantiomer |
|---|---|---|---|---|
| 3 | $-CH_3$ | -CN | $-CH(CH_3)_2$ | 202 |
| 4 | $-CH_3$ | -CN | $-C_2H_5$ | 190 |
| 5 | 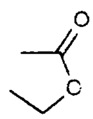 | | $-CH(CH_3)_2$ | 150 |
| 6 | $-CH_3$ | $-NO_2$ | $-C_2H_5$ | 218 |
| 7 | $-CH_3$ | -CN | $-CH_2-CF_3$ | 196 |
| 8 | $-CH_3$ | -CN | $n-C_3H_7$ | 190 |
| 9 | $-CH_3$ | -CN | $-CH(CH_3)_2$ | 226-227 / Enantiomer 1 |
| 10 | $-CH_3$ | -CN | $-CH(CH_3)_2$ | 226-227 / Enantiomer 2 |

In Analogie zur Vorschrift des Beispiels 2 werden die in Tabelle 2 aufgeführten Verbindungen hergestellt:

Tabelle 2:

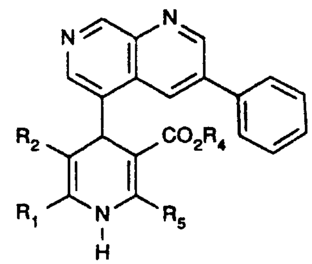

| Bsp.-Nr. | $R^1$ | $R^2$ | $R^4$ | $R^5$ | F°C / $R_f$ / Enantiomer |
|---|---|---|---|---|---|
| 11 | $-CH_3$ | -CN | $-CH(CH_3)_2$ | $-CH_3$ | 192 |
| 12 |  | 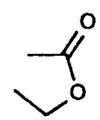 | $-C_2H_5$ | $-CH_3$ | 248 |
| 13 | $-CH_3$ | $-NO_2$ | $-C_2H_5$ | $-CH_3$ | 258 |
| 14 | $-CH_3$ | -CN | $n-C_3H_7$ | $-CH_3$ | 212 |
| 15 |  | 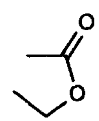 | $-CH(CH_3)_2$ | $-CH_3$ | 160 |
| 16 | $-CH_3$ | $-CO_2-CH(CH_3)_2$ | $-(CH_2)_2OCH_3$ | $-CH_3$ | 193 |

<u>Fortsetzung Tabelle 2:</u>

| Bsp.-Nr. | $R^1$ | $R^2$ | $R^4$ | $R^5$ | F°C / $R_f$ / Enantiomer |
|---|---|---|---|---|---|
| 17 | $-CH_3$ | $-NO_2$ | $-CH(CH_3)_2$ | $-CH_3$ | 255 |
| 18 | | 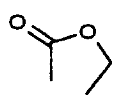 | $-CH_3$ | $-CH_3$ | 250 |
| 19 | $-CH_3$ | $-NO_2$ | $-CH_3$ | $-CH_3$ | 255 |
| 20 | $-CH_3$ | -CN | $n-C_3H_7$ | $-CH_3$ | 224 / (+)-Enantiomer |
| 21 | $-CH_3$ | -CN | $-CH(CH_3)_2$ | $-CH_3$ | Öl / (+)-Enantiomer |
| 22 | $-CH_3$ | -CN | $-CH(CH_3)_2$ | $-CH_3$ | Öl / (-)-Enantiomer |
| 23 | $-CH_3$ | -CN | $n-C_3H_7$ | $-CH_3$ | 219 / (-)-Enantiomer |
| 24 | $-CH_3$ | -CN | $-CH_2-CF_3$ | $-CH_3$ | 199 |
| 25 | $-CH_3$ | -CN | $-C_2H_5$ | $-CH_3$ | 252 |

## Patentansprüche

**1.** Dihydropyridine der allgemeinen Formel (I)

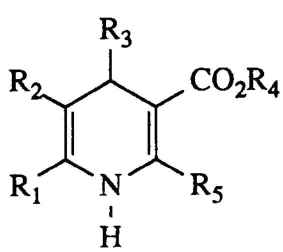

(I),

in welcher

$R^1$ und $R^5$ gleich oder verschieden sind und für geradkettiges oder verzweigtes Alkyl mit bis zu 8 Kohlenstoffatomen stehen,

$R^2$ für geradkettiges oder verzweigtes Alkoxycarbonyl mit bis zu 6 Kohlenstoffatomen steht, das gegebenenfalls durch geradkettiges oder verzweigtes Alkoxy mit bis zu 4 Kohlenstoffatomen substituiert ist,
für Nitro oder Cyano steht,

oder

$R^1$ und $R^2$ gemeinsam einen Lactonring der Formel

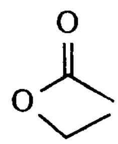

bilden,

$R^3$ für einen heterocyclischen Rest der Formel

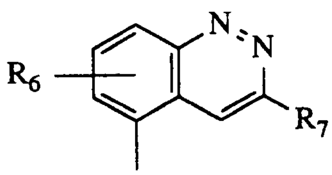

oder

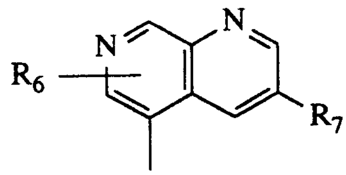

steht,
worin

$R^6$ Wasserstoff, Halogen oder geradkettiges oder verzweigtes Alkyl oder Alkoxy mit jeweils bis zu 8 Kohlenstoffatomen bedeutet,

$R^7$ Aryl mit 6 bis 10 Kohlenstoffatomen bedeutet, das gegebenenfalls bis zu 2-fach gleich oder verschieden durch Halogen, Nitro, Cyano, Trifluormethyl, Trifluormethoxy, Trifluormethylthio, geradkettiges oder verzweigtes Alkyl, Alkoxy oder Alkoxycarbonyl mit jeweils bis zu 8 Kohlenstoffatomen oder Carboxy substituiert ist, oder
Pyridyl oder Thienyl bedeutet,

$R^4$ für Wasserstoff steht, oder
für geradkettiges oder verzweigtes Alkyl, Alkenyl, Alkadienyl oder Alkinyl mit jeweils bis zu 10 Kohlenstoffatomen steht, die gegebenenfalls bis zu 2-fach gleich oder verschieden durch Trifluormethyl, Halogen, Hydroxy, Carboxy, Cyano, Nitro, Phenoxy oder durch geradkettiges oder verzweigtes Alkylthio, Alkoxy, Alkoxycarbonyl, Acyl oder Acyloxy mit jeweils bis zu 8 Kohlenstoffatomen oder durch Phenoxy oder Phenyl substituiert sind, wobei die letzteren ihrerseits bis zu 2-fach gleich oder verschieden durch Halogen oder durch geradkettiges oder verzweigtes Alkyl oder Alkoxy mit jeweils bis zu 6 Kohlenstoffatomen substituiert sein können

und deren physiologisch unbedenklichen Salze.

**2.** Verbindungen der allgemeinen Formel (I) gemäß Anspruch 1, in welcher

$R^1$ und $R^5$ gleich oder verschieden sind und für geradkettiges oder verzweigtes Alkyl mit bis zu 6 Kohlenstoffatomen stehen,

$R^2$ für geradkettiges oder verzweigtes Alkoxycarbonyl mit bis zu 4 Kohlenstoffatomen steht, das gegebenenfalls durch geradkettiges oder verzweigtes Alkoxy mit bis zu 3 Kohlenstoffatomen substituiert ist,
für Nitro oder Cyano steht,

oder

R¹ und R² gemeinsam einen Lactonring der Formel

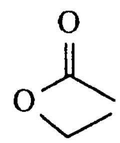

bilden,

$R^3$ für einen heterocyclischen Rest der Formel

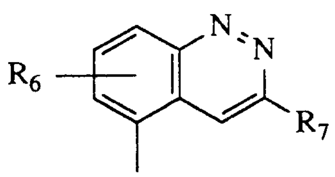

oder

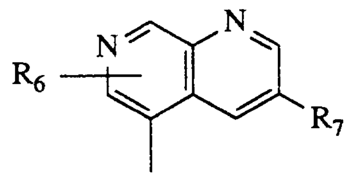

steht,

worin

$R^6$ Wasserstoff, Fluor, Chlor oder geradkettiges oder verzweigtes Alkyl oder Alkoxy mit jeweils bis zu 2 Kohlenstoffatomen bedeutet,

$R^7$ Phenyl bedeutet, das gegebenenfalls durch Fluor, Chlor, Nitro, Cyano, Trifluormethyl oder durch geradkettiges oder verzweigtes Alkyl oder Alkoxy mit jeweils bis zu 6 Kohlenstoffatomen substituiert ist,

$R^4$ für Wasserstoff oder für geradkettiges oder verzweigtes Alkyl oder Alkenyl mit jeweils bis zu 8 Kohlenstoffatomen steht, die gegebenenfalls durch Trifluormethyl, Fluor, Chlor, Hydroxy, Carboxy, Cyano, Nitro oder durch geradkettiges oder verzweigtes Alkylthio, Alkoxy, Alkoxycarbonyl, Acyl oder Acyloxy mit jeweils bis zu 6 Kohlenstoffatomen oder durch Phenoxy oder Phenyl substituiert sind.

**3.** Verbindungen der allgemeinen Formel (I) gemäß Anspruch 1, in welcher

$R^1$ und $R^5$ gleich oder verschieden sind und für geradkettiges oder verzweigtes Alkyl mit bis zu 4 Kohlenstoffatomen stehen,

$R^2$ für geradkettiges oder verzweigtes Alkoxycarbonyl mit bis zu 3 Kohlenstoffatomen oder Methoxyethoxycarbonyl steht, oder für Nitro oder Cyano steht,

oder

$R^1$ und $R^2$ gemeinsam einen Lactonring der Formel

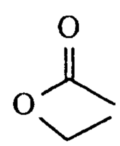

bilden,

$R^3$ für einen heterocyclischen Rest der Formel

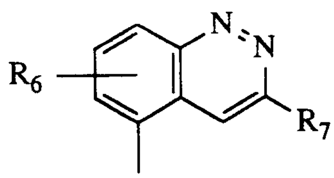 oder 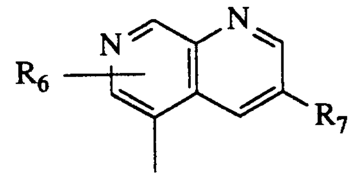

steht,
worin

$R^6$ Wasserstoff, Chlor oder Methyl bedeutet,

$R^7$ Phenyl bedeutet, das gegebenenfalls durch Fluor, Chlor, Nitro, Trifluormethyl oder durch geradkettiges oder verzweigtes Alkyl oder Alkoxy mit jeweils bis zu 4 Kohlenstoffatomen substituiert ist,

$R^4$ für Wasserstoff steht, oder
für geradkettiges oder verzweigtes Alkyl mit bis zu 6 Kohlenstoffatomen steht, das gegebenenfalls durch Trifluormethyl, Hydroxy, Carboxy, Cyano oder durch geradkettiges oder verzweigtes Alkoxycarbonyl, Alkoxy oder Acyloxy mit jeweils bis zu 4 Kohlenstoffatomen substituiert ist.

**4.** Verfahren zur Herstellung von Verbindungen der allgemeinen Formel (I) gemäß Anspruch 1, dadurch gekennzeichnet, daß man für den Fall, daß $R^1$ und $R^2$ nicht gemeinsam einen Lactonring bilden,
[A] Aldehyde der allgemeinen Formel (II)

$R^3$-CHO (II),

in welcher
$R^3$ die in Anspruch 1 angegebene Bedeutung hat,
zunächst mit Acetessigestern der allgemeinen Formel (III)

$R^5$-CO-$CH_2$-$CO_2$-$R^4$ (III),

in welcher
$R^4$ und $R^5$ die in Anspruch 1 angegebene Bedeutung haben,
gegebenenfalls unter Isolierung der entsprechenden Ylidenverbindungen der allgemeinen Formel (IV)

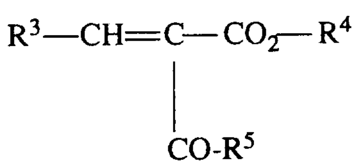 (IV),

in welcher
$R^3$, $R^4$ und $R^5$ die in Anspruch 1 angegebene Bedeutung haben,
umsetzt, und anschließend entweder mit Verbindungen der allgemeinen Formel (V)

$R^1$-CO-$CH_2$-$R^2$ (V),

in welcher
$R^1$ und $R^2$ die oben angegebene Bedeutung haben,
in Anwesenheit von Ammoniak oder Ammoniumsalzen, oder direkt mit Aminoderivaten der allgemeinen Formel (VI)

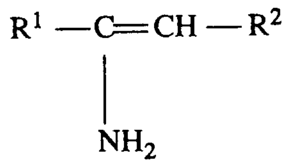

(VI),

in welcher

$R^1$ und $R^2$ die oben angegebene Bedeutung haben,

gegebenenfalls in Gegenwart von inerten organischen Lösemitteln umsetzt,

oder

[B] die Aldehyde der allgemeinen Formel (II) zunächst mit den Verbindungen der allgemeinen Formel (V), gegebenenfalls unter Isolierung der Ylidenverbindungen der allgemeinen Formel (VII)

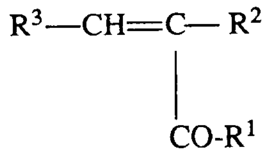

(VII),

in welcher

$R^1$, $R^2$ und $R^3$ die oben angegebene Bedeutung haben,

umsetzt und in einem nächsten Schritt mit den oben aufgeführten Verbindungen der allgemeinen Formel (III) in inerten Lösemitteln, in Anwesenheit von Ammoniak oder Ammoniumsalzen oder direkt mit Enaminocarbonsäurederivaten der allgemeinen Formel (VIII)

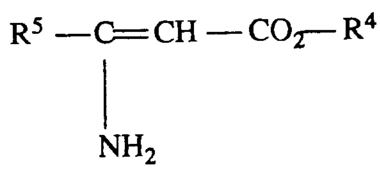

(VIII),

in welcher

$R^4$ und $R^5$ die oben angegebene Bedeutung haben,

umsetzt,

oder im Fall, daß $R^1$ und $R^2$ gemeinsam einen Lactonring bilden,

[C] zunächst nach denen unter [A] und [B] aufgeführten Methoden, Verbindungen der allgemeinen Formel (Ia)

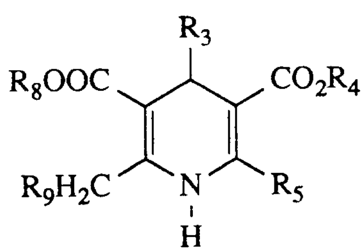

(Ia),

in welcher

$R^3$, $R^4$ und $R^5$ die oben angegebene Bedeutung haben,

$R^8$ für einen $C_1$-$C_6$-Alkyl-Rest steht

und

$R^9$ für eine Abgangsgruppe wie beispielsweise Chlor oder Acetoxy steht,

herstellt und nach bekannten Verfahren einen säure- oder basenkatalysierten Ringschluß anschließt, oder im Fall, daß $R^4$ nicht Wasserstoff bedeutet,

[D] Verbindungen der allgemeinen Formel (I), in welcher $R^1$, $R^2$, $R^3$, $R^4$ und $R^5$ die oben angegebene Bedeutung haben und $R^4$ für Wasserstoff steht, gegebenenfalls über ein reaktives Säurederivat, mit den entsprechenden Alkoholen umsetzt, wobei durch Einsatz der enantiomerenreinen Carbonsäuren ($R^4$ = H) die entsprechenden Enantiomere der Ester erhalten werden.

**5.** Aldehyde der allgemeinen Formel (II)

$R^3$-CHO (II),

in welcher

$R^3$ die in Anspruch 1 angegebene Bedeutung hat.

**6.** Verfahren zur Herstellung von Aldehyden der allgemeinen Formel (II) gemäß Anspruch 5, dadurch gekennzeichnet, daß man für den Fall, daß $R^3$ für den Rest der Formel

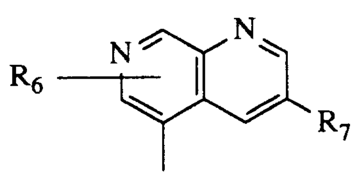

steht,

a) substituierte Pyridine der allgemeinen Formel (IX)

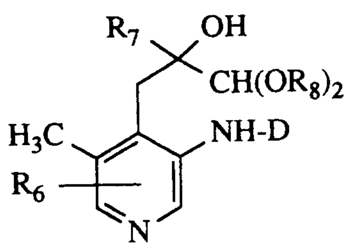

(IX),

in welcher

$R^6$ und $R^7$ die in Anspruch 4 angegebene Bedeutung haben,

D für eine Schutzgruppe steht

und

$R^8$ für geradkettiges oder verzweigtes Alkyl mit bis zu 4 Kohlenstoffatomen steht,

zunächst mit Protonensäuren, vorzugsweise Salzsäure und anschließender Hydrierung zu den Verbindungen der allgemeinen Formel (X)

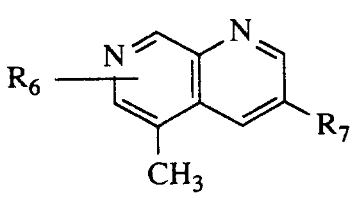

(X),

in welcher

$R^6$ und $R^7$ die oben angegebene Bedeutung haben,

in inerten Lösemitteln cyclisiert und in einem letzten Schritt in einem organischen Lösemittel oder Naphthalin die Methylgruppe oxidiert und im Fall, daß $R^3$ für den Rest der Formel

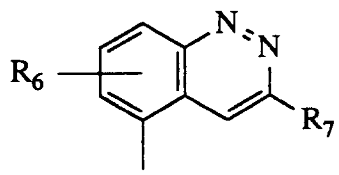

steht,

b) Verbindungen der allgemeinen Formel (XI)

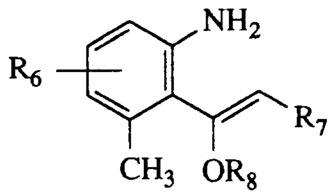

(XI),

in welcher

$R^6$, $R^7$ und $R^8$ die oben angegebene Bedeutung haben,

nach Hydrolyse, über die diazotierte Stufe ($NH_2$ -> $N_2^+$) zu Verbindungen der allgemeinen Formel (XII)

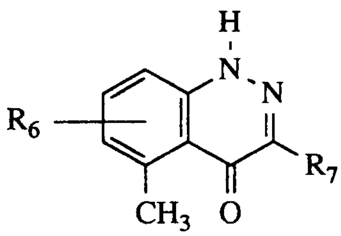

(XII),

in welcher

$R^6$ und $R^7$ die oben angegebene Bedeutung haben,

cyclisiert, in einem zweiten Schritt mit $PCl_5/POCl_3$ in Verbindungen der allgemeinen Formel (XIII)

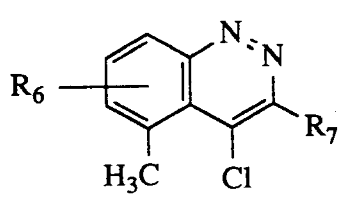

(XIII),

in welcher

$R^6$ und $R^7$ die oben angegebene Bedeutung haben

überführt, hydriert und abschließend die Methyl-Gruppe in inerten Lösemitteln oxidiert.

**7.** Verbindungen der allgemeinen Formel (IX)

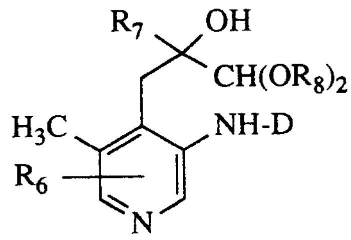

(IX),

in welcher

| | |
|---|---|
| $R^6$ und $R^7$ | die in Anspruch 4 angegebene Bedeutung haben, |
| D | für eine Schutzgruppe steht und |
| $R^8$ | für niederes Alkyl steht. |

**8.** Arzneimittel enthaltend mindestens eine Verbindung der allgemeinen Formel (I) gemäß Anspruch 1.

**9.** Verfahren zur Herstellung von Arzneimitteln, dadurch gekennzeichnet, daß man Verbindungen der allgemeinen Formel (I) gemäß Anspruch 1, gegebenenfalls unter Verwendung von üblichen Hilfs- und Trägerstoffen in eine geeignete Applikationsform überführt.

**10.** Verwendung von Verbindungen der allgemeinen Formel (I) gemäß Anspruch 1 bei Herstellung von Arzneimitteln zur Bekämpfung von Krankheiten.

Europäisches Patentamt

# EUROPÄISCHER RECHERCHENBERICHT

Nummer der Anmeldung

EP 93 10 0649

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 452 712 (BAYER AG)<br>* das ganze Dokument *<br>--- | 1,5,8 | C07D401/10<br>C07D471/04<br>C07D491/04<br>A61K31/44<br>A61K31/50 |
| A | EP-A-0 186 028 (BAYER AG)<br>* Seite 1 - Seite 6, Zeile 17 *<br>* Seite 12, Zeile 16 - Seite 14, Zeile 10 * | 1,8 | |
| D | & DE-A-3 447 169<br>--- | | |
| A | US-A-4 284 634 (YOSHINARI SATU)<br>* Spalte 1 - Spalte 4, Zeile 28 * | 1,8 | |
| D | & DE-A-2 629 892<br>--- | | |
| A | US-A-4 038 399 (FRIEDRICH BOSSERT ET AL.)<br>* Spalte 1 - Spalte 4 *<br>--- | 1,8 | |
| A,D | NATURE<br>Bd. 303, 9. Juni 1983, UK<br>Seiten 535 - 537<br>M. SCHRAMM ET AL. 'Novel Dihydropyridines with positive inotropic action through activation of Ca++ Channels'<br>* das ganze Dokument *<br>--- | 1,8 | RECHERCHIERTE SACHGEBIETE (Int. Cl.5)<br>C07D |
| A | COLLECTION OF CZECHOSLOVAK CHEMICAL COMMUNICATIONS<br>Bd. 57, Nr. 1, Januar 1992, PRAGUE<br>Seiten 169 - 178<br>P.G. BARALDI ET AL. 'Synthesis and Calcium antagonist activity of Dialkyl 1,4-Dihydro-2,6-Dimethyl-4-(nitrogenous Heteroaryl)-3,5-Pyridine Dicarboxylates'<br>* das ganze Dokument *<br>----- | 1,8 | |

Der vorliegende Recherchenbericht wurde für alle Patentansprüche erstellt

| Recherchenort | Abschlußdatum der Recherche | Prüfer |
|---|---|---|
| BERLIN | 25 MAI 1993 | KYRIAKAKOU G. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)